(19) Europäisches Patentamt
European Patent Office
Office européen des brevets

(11) **EP 1 907 962 B1**

(12) # EUROPEAN PATENT SPECIFICATION

(45) Date of publication and mention
of the grant of the patent:
**14.01.2009 Bulletin 2009/03**

(51) Int Cl.:
***G06F 19/00*** (2006.01)

(21) Application number: **06754015.3**

(22) Date of filing: **31.05.2006**

(86) International application number:
**PCT/EP2006/005196**

(87) International publication number:
**WO 2007/006373 (18.01.2007 Gazette 2007/03)**

(54) **METHOD FOR CALCULATION OF STATISTICAL CHARACTERISTICS FOR A SET OF GENE EXPRESSION VALUES**

VERFAHREN ZUR BERECHNUNG STATISTISCHER KENNGRÖSSEN FÜR EINE MENGE VON GENEXPRESSIONSWERTEN

METHODE DE CALCUL DE CARACTERISTIQUES STATISTIQUES D'UN ENSEMBLE DE VALEURS D'EXPRESSION GENIQUE

(84) Designated Contracting States:
**DE GB**

(30) Priority: **12.07.2005 EP 05015085**

(43) Date of publication of application:
**09.04.2008 Bulletin 2008/15**

(73) Proprietors:
• **Thurner, Stefan**
**1080 Wien (AT)**
• **Wick, Nikolaus**
**1190 Wien (AT)**
• **Biely, Christoly**
**3411 Weidling (AT)**
• **Stokic, Dejan**
**1090 Wien (AT)**

(72) Inventors:
• **Thurner, Stefan**
**1080 Wien (AT)**
• **Wick, Nikolaus**
**1190 Wien (AT)**
• **Biely, Christoly**
**3411 Weidling (AT)**
• **Stokic, Dejan**
**1090 Wien (AT)**

(74) Representative: **Vossius & Partner**
**Siebertstraße 4**
**81675 München (DE)**

(56) References cited:
**WO-A-01/84139**

• **PAN W ET AL: "A mixture model approach to detecting differentially expressed genes with microarray data" FUNCTIONAL AND INTEGRATIVE GENOMICS, SPRINGER,, DE, vol. 3, no. 3, July 2003 (2003-07), pages 117-124, XP002350990 ISSN: 1438-793X**
• **YAN X ET AL: "Detecting differentially expressed genes by relative entropy" JOURNAL OF THEORETICAL BIOLOGY, ACADEMIC PRESS, LONDON, GB, vol. 234, no. 3, 7 June 2005 (2005-06-07), pages 395-402, XP004797694 ISSN: 0022-5193**
• **PAN W: "A comparative review of statistical methods for discovering differentially expressed genes in replicated microarray experiments" BIOINFORMATICS, OXFORD UNIVERSITY PRESS, OXFORD,, GB, vol. 18, no. 4, April 2002 (2002-04), pages 546-554, XP002350991 ISSN: 1367-4803**

**Description**

**Field of the invention**

The invention is concerned with the field of analysis of data resulting from gene profiling experiments.

**Background of the invention**

In gene expression profiling, large numbers of probes are commonly hybridized by RNA extracted from cells or tissue to be analyzed. The method allows the parallel determination of expression levels measured in terms of RNA abundance of a multitude of genes. An inherent problem of the method is a certain variations of the detected expression levels. This variation is caused by the measurement method or by factors inherent to the analyzed sample. This constitutes a problem, because in analyzing the samples, one must determine which changes in expression level (e.g. upon application of a stimulus) are significant and which merely reflect statistical variation.

It is also important to detect those genes which are very unlikely to belong to statistical noise but constitute biological relevance. The proposed method of the present invention is especially designed to single out these genes in a statistically consistent and homogenous way over a large range of expression levels.

Commonly, results of gene profiling experiments are often displayed in an X-Y scatter graph. The X axis represents the expression level in condition 1 (e.g., control), whereas the Y axis represents the expression level in condition 2 (e.g., induced with a stimulus). The X and Y axis may also represent different cell lines or tissues (e.g., diseased versus healthy tissue) that are to be compared.

Each gene is represented on the plot by a single dot. Genes that are expressed equally in both conditions or tissues will be located on a straight line crossing the intersection of both axes and having a 45° angle. Dots above the line correspond to genes that are more expressed in the condition reflected by the Y axis, whereas dots below the line correspond to genes that are more expressed in the condition reflected by the X axis.

In the state of the art of the analysis of gene profiling data, thresholds are used to determine which genes might be over-expressed at relevant levels. For instance, one may set the threshold at a factor of 2 or more so that all genes whose expression level are less than 2 fold compared to the other are considered insignificant changes. Alternatively, also standard t-tests and variants of it are in use.

WO 01/84139 describes an example of the existing methods. In more detail, "Significance Analysis of Microarrays". (SAM) represents a modified t-statistics, where a fudge factor (which modifies the t-statistics) is calculated by a permutation estimation approach. In SAM, a grouping of genes is used to calculate just that fudge factor. In particular, grouping is done for a parameter. Within those groups another parameter is computed. These parameters of all groups are then combined into a function (e.g. a variance estimator) which can be optimized (minimized) with a specific fudge factor. In short, in SAM, a global factor (not group specific) minimizing the heterogenity of deviations of single experiments is calculated via a grouping procedure.

The 2-fold (or x-fold) deviation is often used because at these levels experimental checks as e.g. real-time PCR work reasonably well.

The 2-fold (or x-fold) deviation can be marked directly in that graph (often in double logarithmic form) as two lines in parallel above and below the said line. Dots located inside of the area delineated by these two lines will be below the 2-fold (x-fold) deviation threshold and will be considered genes that have not significantly changed.

The inventors state that the definition of the x-fold deviation is highly critical to the identification of genes associated with the different conditions. For instance, when a cancerous tissue and a healthy tissue of the same type are compared, many changes in expression levels are detected. The physiologically most important of these may not be the strongest change in expression level (e.g., a more than 30-fold change), but may very well be a minor change in a gene that drives or reflects the most important physiological changes. At the same time, a statistically insignificant change would merely reflect clutter and have no informational value and thus not merit further time-consuming investigation.

**Summary of the invention**

The object of the present invention is to provide an improved method of thresholding in a method for calculation of statistical characteristics for a set of gene expression values. This object is achieved with the features of the claims.

Generally, the present invention relates to methods of calculating distribution functions and their characterization, such as e.g. their standard deviation for a set of gene expression values. More specifically, the invention provides a method for calculation of distributions and their characteristics such as e.g. their standard deviation values for a set of gene expression value pairs $(X_n|Y_n)$, comprising the steps of a) grouping pairs and b) calculating the distribution characteristics such as the standard deviation for each group.

In a preferred method of determining the significance level of changes in gene expression patterns according to the present invention, the gene expression values $(X_n \mid Y_n)$ are first grouped according to their expression level. This is preferably done by rotating the expression levels displayed as dots on a. X-Y-graph as described above by 45°. Each gene i, i.e. pair $(X_i, Y_i)$ will be rotated by applying the rotation matrix M on that pair: $(X_i', Y_i') = M(X_i, Y_i)$, where M is a 2x2 matrix whose components are given by $M_{11} = \cos(\varphi)$, $M_{12} = \sin(\varphi)$, $M_{21} = -\sin(\varphi)$ and $M_{22} = -\cos(\varphi)$, where $\varphi$ is the desired rotation angle of -45°. The line on which all unchanged genes are displayed is now falling on the Y axis. Alternatively, a clockwise rotation is performed by setting $\varphi = +45°$.

After rotation, the space around the Y axis is preferably divided by lines parallel to the X axis. More preferably, these lines are equally spaced. As an alternative, it is preferred to use a variable spacing, such that all the bands contain the same number of genes in it. Segmentation or grouping, respectively, is preferably achieved by providing thresholds $T_n$ that are parallel to the X axis and which divide the data into groups $G_n$. Preferably, all value pairs of a group $G_n$ satisfy $T_n < \sqrt{(X_n^2 + Y_n^2)} < T_{(n+1)}$, before rotation, or equivalently $T_n < (Y_n') < T_{(n+1)}$ after rotation. The number of genes within the bands has to be large enough to ensure reasonable estimation of the distribution functions within these bands (preferably more then 10, more preferably more then 100, and most preferably more than 1000). Also preferably, about 20-30, and more preferably about 50-100 lines, i.e., groups, are used. Alternative ways of grouping are encompassed by the present invention which result in a defined number of groups.

In a subsequent step, the distribution functions and their characteristics such as e.g. the standard deviation and eventually higher moments and functions thereof are calculated. The distribution function f(X) is computed by means of histograms of all genes within one group after rotation. (Histograms can be soothed or non-smoothed, can be approximated by a Gaussian distribution or taken as raw data. The binning for obtaining the histograms is a free parameter to the procedure and has to be chosen appropriately, i.e. consistent with the normalization scheme used for the gene expression data, and such that the number of populated bins is neither to low, nor to high and noisy). The characteristic moments are then computed either directly by assuming a Gaussian distribution (e.g. mean, standard deviation, skewness, or kurtosis) or by computing the moments with: $\text{moment}_1 = \int f(X)X \, dX$, $\text{moment}_2 = \int f(X)X^2 \, dX$, $\text{moment}_3 = \int f(X)X^3 \, dX$), etc. for each group separately. This is done by taking all values in a group and calculating these statistical values, preferably the standard deviation, or second moment for that group. As mentioned above, groups are chosen such that there will be sufficiently many items for reliable computation of these statistical characteristics.

The standard deviation for N samples (genes) in a group is preferably calculated with the following algorithm. $X_i'$ are the values after rotation:

$$Std(X')^2 = \frac{1}{(N-1)} \bullet \sum_{i=1}^{N} \left(X'_i - mean(X'_i)\right)^2$$

These segmentally calculated characteristics, e.g. standard deviations or other statistical measures are now used as the basis for the delineation of insignificant changes versus significant changes in gene expression. If desired, the standard deviation or other measures for each group may be plotted along the X axis of the graph, resulting in an approximately vase-shaped contour around the Y axis. This is because the distribution functions of genes with lower expression levels will be wider than that of genes with higher expression levels so that e.g. the standard deviation curve in the lower part of the graph will the broader than in the upper part of the graph.

Optionally, the statistical characteristics e.g. the standard deviation curve may be smoothed. This may be of advantage because by segmenting the data, fewer data are available for each segment to calculate the standard deviation. It is therefore possible that the number of data for some segments is too small to correctly calculate the standard deviation. This may be overcome by smoothing e.g. the standard deviation curve. A number of methods, such as calculation of the standard deviation by using an adjusted mean of n adjacent standard deviation values could be used.

Further optionally, the so obtained statistical characteristics for the individual groups such as e.g. the standard deviation curve may be rotated back by a 45° angle to provide the usual gene expression profiling result graph, but with the improved standard deviation curve surrounding the central line.

By using the improved method of calculating standard deviations, the detection of significantly changed genes is now made much easier, as false negative identifications (in cases of larger expression levels or in the upper right corner of the graph, respectively) are avoided, as well as false positive identifications (in the lower left corner of the graph or for genes with lower expression levels, respectively).

## Brief description of the Figures

Fig. 1  shows a X-Y plot representation of expression levels in a gene profiling experiment;

Fig. 2  shows the direction of rotation of the expression values according to a preferred embodiment of the present invention;

Fig. 3  shows the data set of Fig. 1 after rotation by 45° for the embodiment of Fig. 2;

Fig. 4  shows how segmentation is achieved by providing thresholds $T_n$ that are parallel to the X axis and which divide the data into a plurality of groups $G_n$;

Fig. 5  is a flow diagram of a preferred embodiment of the present invention;

Fig. 6  shows the BEC signal intensity vs. LEC signal intensity for a larger number of genes;

Fig. 7  shows the mRNA abundance vs. the BEC/LEC ratio; for the example of Fig. 6;

Fig. 8  shows the relative mRNA abundance vs. the BEC/LEC ratio after forming equi-populated groups; for the example of Fig. 6;

Fig. 9  shows the BEC/LEC ratio vs. the relative mRNA abundance for the example of Fig. 6, however, in comparison to Fig. 8, different regions for the standard deviation are shown;

Fig. 10  shows the BEC signal intensity vs. LEC signal intensity for the example of Fig. 8 with standard deviation regions indicated; and '

Fig. 11  shows the result of the standard t-test applied on the data set of Fig. 6.

## Detailed description of the invention

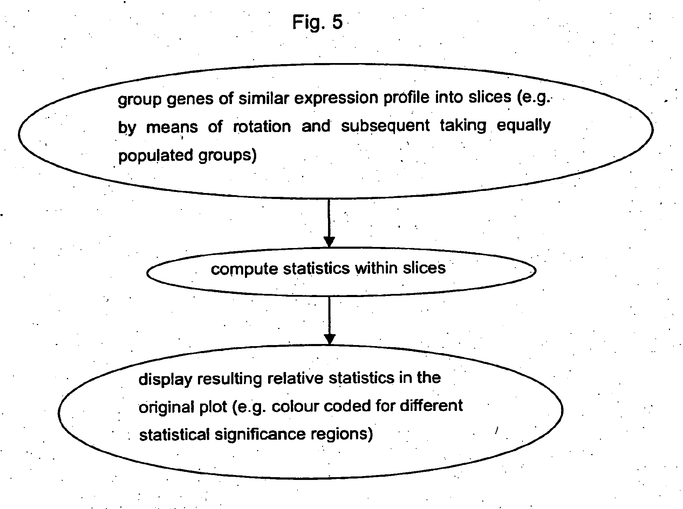

Fig. 5 shows a flow diagram of the preferred embodiment of the present invention. According to the first step shown in Fig. 5, genes of similar expression profiles are grouped into slices. As described above, this is preferably done by rotation of the data and subsequent taking equally populated groups. In the next step, the statistics within slices are calculated, as described above. Finally, the resulting relative statistics are displayed in the original plot. Preferably, different statistical significance regions are color coded.

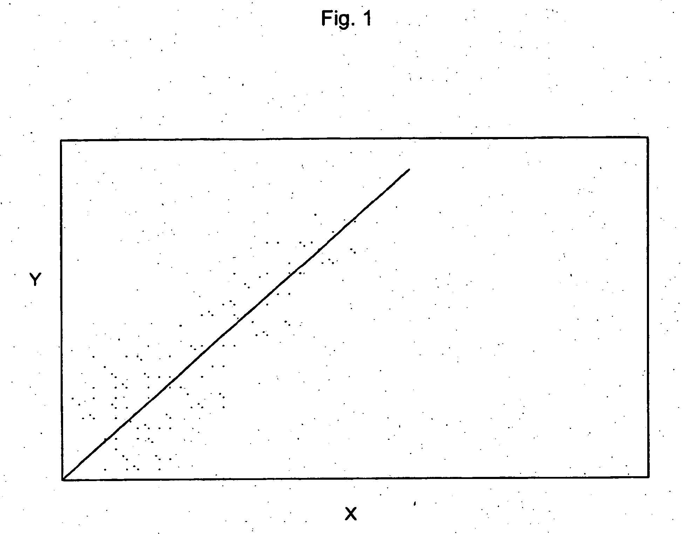

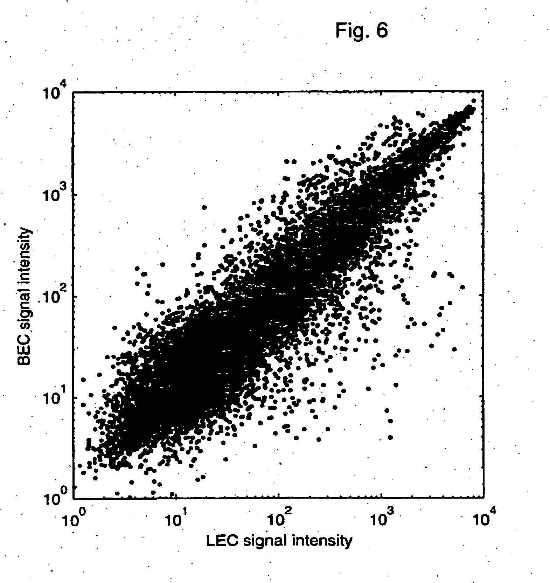

Fig. 1 shows an X-Y plot representation of expression levels in a gene profiling experiment. The X axis denotes expression values e.g., derived from lymphatic endothelial cells (LEC), whereas the Y axis denotes expression values e.g., derived from blood endothelial cells (BEC). Each gene is marked as a dot on the graph, with the position of the dot determined by the expression level of the gene. The straight line at 45 degrees denotes the locations of genes that are expressed equally in both cell lines, i.e., where there is no difference in expression level. Fig. 6 relates to the same example, showing BEC signal intensity vs. LEC signal intensity for a larger number of genes.

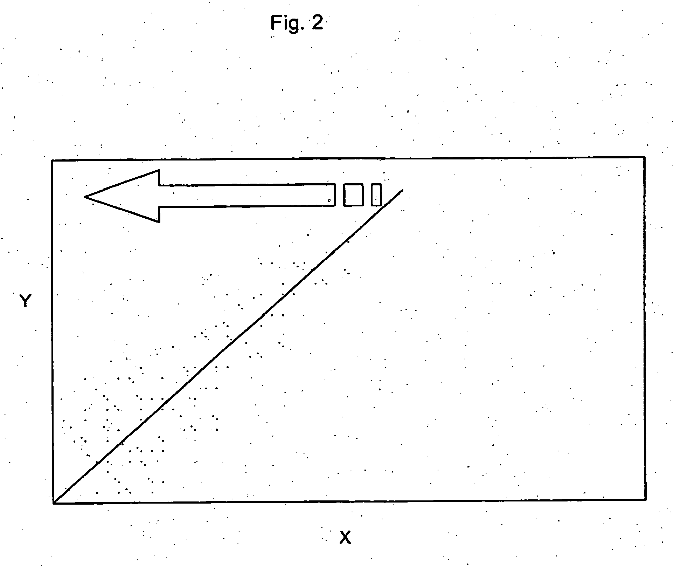

Fig. 2 shows the direction of rotation of the expression values. By rotating the data set according to a preferred embodiment of the present invention counterclockwise by 45°, the line denoting equal expression of genes in both cell lines now coincides with the Y axis of the graph.

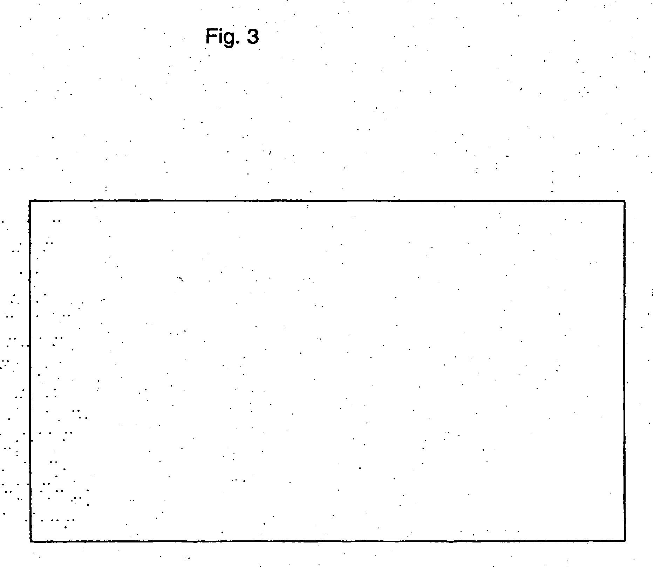

Fig. 3 shows the data set after rotation by 45°. The data are now centered around the Y axis.

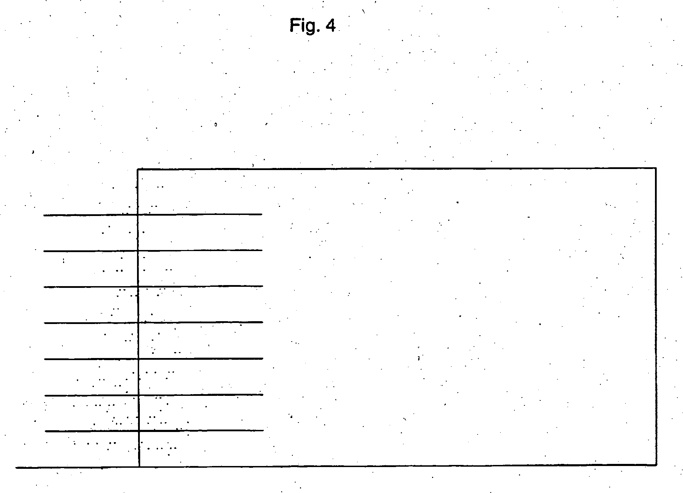

Fig. 4 shows how segmentation is achieved by providing thresholds $T_n$ that are parallel to the X axis and which divide the data into groups $G_n$. According to the preferred embodiment shown in Fig. 4, the thresholds are equally spaced from each other. On the basis of such grouped data, the significance values are calculated. After calculation of significance values, the segmented data are preferably be rotated back.

Optionally, values that are located outside of a specified significance threshold, e.g., $2\sigma$, are marked in the graph, e.g. by different (e.g., red) coloring, to enable easy identification.

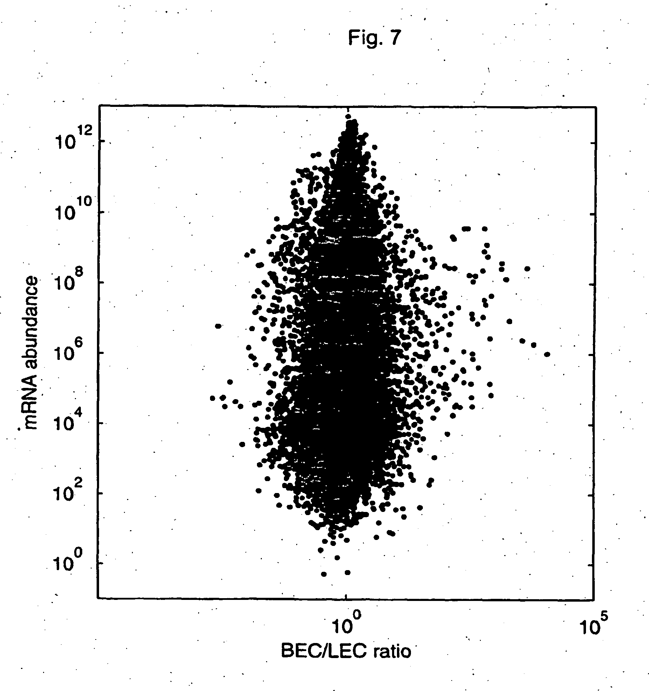

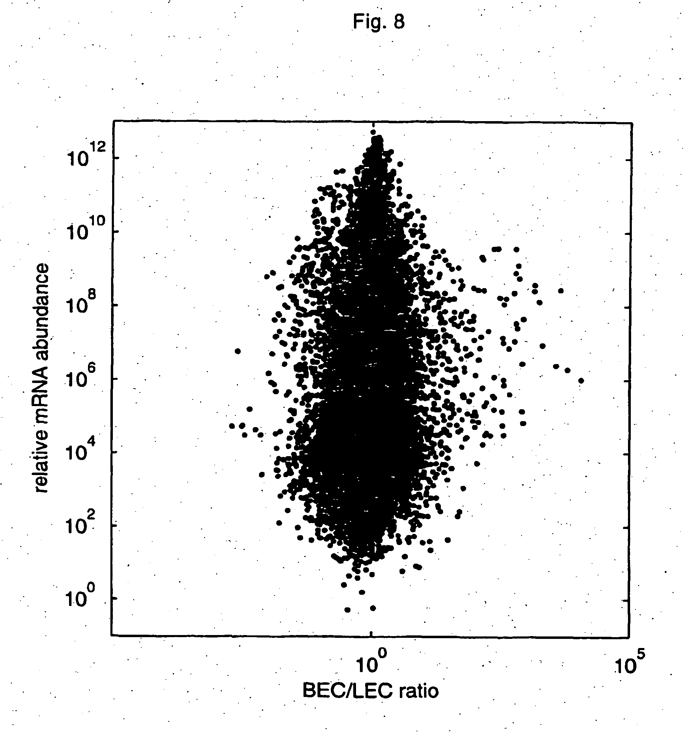

Figs 5 to 9 show a more specific example on to which the method of the present invention was applied. As mentioned above, Fig. 6 shows BEC signal intensity vs. LEC signal intensity for a larger number of genes. For the X and Y axis, the double logarithmic form is used. For this example, Fig. 7 shows the mRNA abundance vs. the BEC/LEC ratio. Furthermore, Fig. 8 shows the relative mRNA abundance vs. the BEC/LEC ratio; for the embodiment of Fig. 6.

As mentioned above, segmentally calculated characteristics, e.g. standard deviations or other statistical measures are used as the basis for the delineation of insignificant changes versus significant changes in gene expression. The standard deviation or other measures for each group are preferably plotted along the X axis of the graph, resulting in an approximately vase-shaped contour around the Y axis.

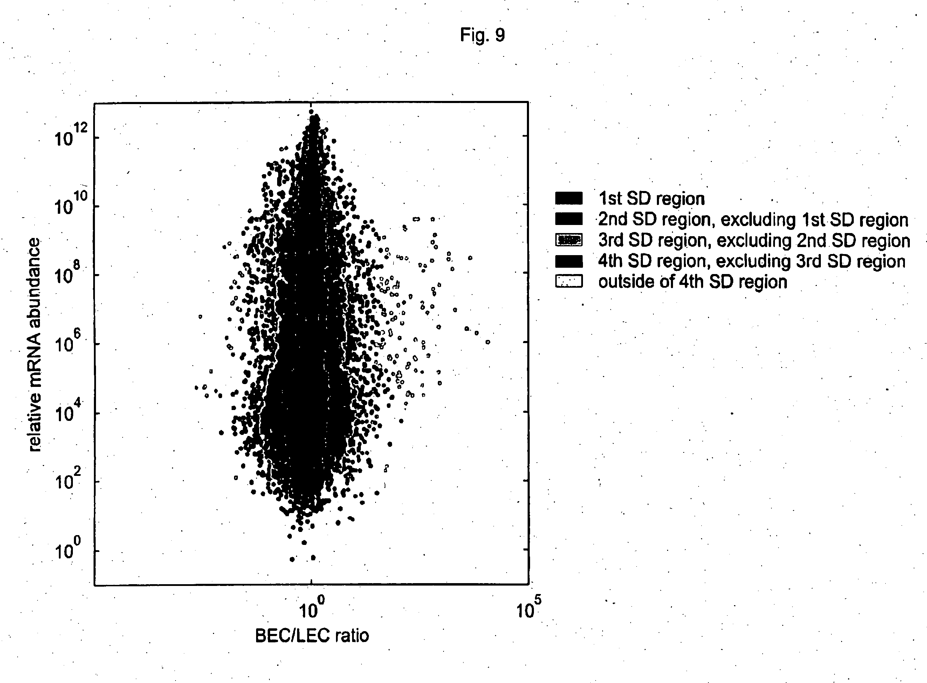

Fig. 9 also shows the BEC/LEC ratio vs. the relative mRNA abundance for the example of Fig. 6. However, in comparison to Fig. 8, different regions for the standard deviation are shown. The central region at a BEC/LEC ratio of about 1 is the first standard deviation (in the Figures "SD") region. With decreasing and increasing BEC/LEC ratios, the following regions follow in this order: second standard deviation region (excluding the first SD region), third standard deviation region (excluding the second SD region), fourth standard deviation region (excluding the third SD region), and finally the remaining data outside the fourth SD region. In order to easily visualize the different regions, the regions are preferably colored differently (in Fig. 9, different grey levels are used to differentiate the various regions).

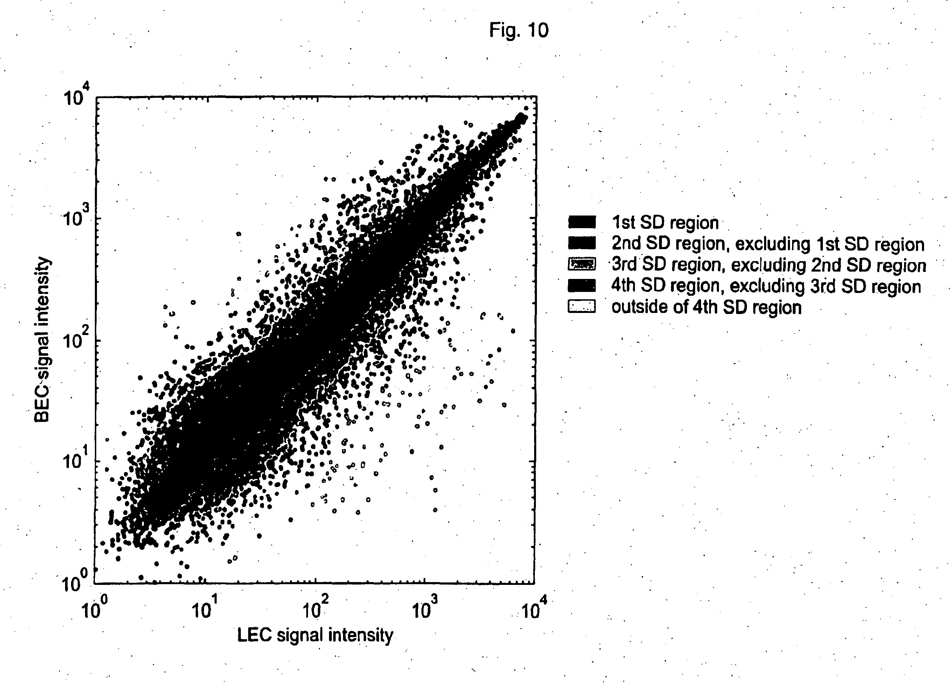

Fig. 10 shows the data after the step of rotating the data back by 45°. Gain, the different standard deviation regions are shown with different colors.

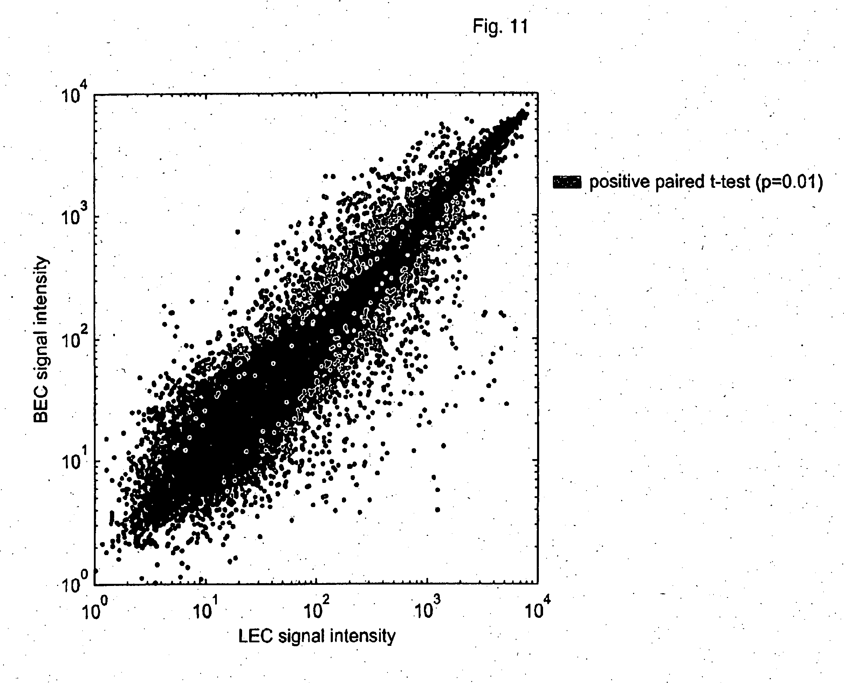

In Fig. 11, the standard prior art approach (so-called t-test) is shown. The lighter colored data plots in this Figure represent the positive paired t-test data for p<0.01. A comparison of Figs. 10 and 11 clearly shows that different genes are identified.

**Claims**

1. Computer-implemented method for identifying differentially expressed genes for a set of gene expression value pairs ($X_n$ | $Y_n$), comprising the steps of:

    a) grouping said set of value pairs into a plurality of groups $G_n$ according to their expression level;
    b) calculating the distribution characteristics for each group thus identifying a subgroup specific significance threshold; and
    c) identifying those genes that are located outside each said specified subgroup significance threshold.

2. The method according to claim 1, wherein step a) comprises the steps of:

    a1) rotating each value pair in a X-Y-representation by a predetermined rotation angle;
    a2) dividing the range within which the value pairs are located into bands.

3. The method of claim 2, wherein in step a2) the bands are equally spaced.

4. The method according to claim 2 or 3, wherein the groups are defined by setting threshold values $T_n$ wherein all value pairs of a group $G_n$ satisfy $T_n < \sqrt{(X^2_n+Y^2_n)} < T_{(n+1)}$, before rotation, or equivalently $T_n < (Y_n') < T_{(n+1)}$ after rotation.

5. The method according to any of claims 1 to 4, wherein the number of groups is about 20-30, more preferably about 50-100.

6. The method according to any of claims 1 to 5, wherein in step b) the distribution characteristics for a group is calculated by calculating the mean of the distribution characteristic of the group $G_n$.

7. The method according to claim 6 wherein the mean is a weighted mean.

8. Method of any of claims 1 to 7, wherein said distribution characteristics are standard deviation values.

9. The method of claim 8, further comprising the step of determining whether a given pair of expression values is located within or outside a predetermined significance threshold.

10. The method of claim 9, further comprising delineation of insignificant changes versus significant changes in gene expression on the basis of said segmentally calculated distribution characteristics.

**Patentansprüche**

1. Computer-implementiertes Verfahren zum Identifizieren differenziell exprimierter Gene für einen Satz von Genexpressions-Wertepaaren ($X_n$ | $Y_n$) mit folgenden Schritten:

    a) Gruppieren des Satzes von Wertepaaren in mehrere Gruppen $G_n$ entsprechend ihrem Expressionsniveau,
    b) Berechnen der Verteilungskenngrößen für jede Gruppe, wodurch eine Untergruppen-spezifische Signifikanzschwelle identifiziert wird, und
    c) Identifizieren jener Gene, die sich außerhalb von jeder der spezifizierten Untergruppen-Signifikanzschwellen befinden.

2. Verfahren nach Anspruch 1, wobei Schritt a) folgende Schritte aufweist:

a1) Drehen jedes Wertepaars in einer XY-Darstellung um einen vorbestimmten Drehwinkel und

a2) Unterteilen des Bereichs, innerhalb dessen sich die Wertepaare befinden, in Bänder.

3. Verfahren nach Anspruch 2, wobei in Schritt a2) die Bänder gleich beabstandet sind.

4. Verfahren nach Anspruch 2 oder 3, wobei die Gruppen definiert werden, indem Schwellenwerte $T_n$ festgelegt werden, wobei alle Wertepaare einer Gruppe $G_n$ $T_n < \sqrt{\left(X_n^2 + Y_n^2\right)} < T_{(n+1)}$ vor der Drehung oder gleichwertig $T_n < (Y_n') < T_{(n+1)}$ nach der Drehung erfüllen.

5. Verfahren nach einem der Ansprüche 1 bis 4, wobei die Anzahl der Gruppen etwa 20 - 30, bevorzugter etwa 50 - 100, beträgt.

6. Verfahren nach einem der Ansprüche 1 bis 5, wobei in Schritt b) die Verteilungskenngrößen für eine Gruppe berechnet werden, indem der Mittelwert der Verteilungskenngröße der Gruppe $G_n$ berechnet wird.

7. Verfahren nach Anspruch 6, wobei der Mittelwert ein gewichteter Mittelwert ist.

8. Verfahren nach einem der Ansprüche 1 bis 7, wobei die Verteilungskenngrößen Standardabweichungswerte sind.

9. Verfahren nach Anspruch 8, welches ferner folgenden Schritt aufweist: Bestimmen, ob sich ein gegebenes Paar von Expressionswerten innerhalb oder außerhalb einer vorbestimmten Signifikanzschwelle befindet.

10. Verfahren nach Anspruch 9, welches ferner folgenden Schritt aufweist: Darstellen nicht signifikanter Änderungen gegenüber signifikanten Änderungen in einer Genexpression auf der Grundlage der segmentell berechneten Verteilungskenngrößen.

## Revendications

1. Procédé implémenté par ordinateur pour identifier des gènes exprimés de manière différentielle pour un ensemble de paires de valeurs d'expression de gène $(X_n|Y_n)$, comprenant les étapes consistant à :

a) répartir ledit ensemble de paires de valeurs sous la forme d'une pluralité de groupes $G_n$ en fonction de leur niveau d'expression ;
b) calculer les caractéristiques de distribution pour chaque groupe en identifiant, en conséquence, un seuil significatif spécifique à un sous-groupe ; et
c) identifier les gènes qui sont situés en dehors de chaque seuil significatif d'un sous-groupe spécifié.

2. Procédé selon la revendication 1, dans lequel l'étape a) comprend les étapes consistant à :

a1) faire basculer chaque paires de valeurs dans une représentation de type X-Y selon un angle de rotation prédéterminé ; et
a2) diviser la plage, dans laquelle se trouvent les paires de valeurs, sous la forme de bandes.

3. Procédé selon la revendication 2, dans lequel, à l'étape a2), les bandes sont espacées de manière équidistante.

4. Procédé selon la revendication 2 ou 3, dans lequel les groupes sont définis en fixant des valeurs de seuil $T_n$, dans lesquels toutes les paires de valeurs d'un groupe $G_n$ satisfont la relation $T_n < \sqrt{(X_n^2 + Y_n^2)} < T_{(n+1)}$ avant rotation ou, de manière équivalente, la relation $T_n < (Y_n') < T_{(n+1)}$ après rotation.

5. Procédé selon l'une quelconque des revendications 1 à 4, dans lequel le nombre de groupes est d'environ 20 à 30, mieux encore, d'environ 50 à 100.

6. Procédé selon l'une quelconque des revendications 1 à 5, dans lequel, à l'étape b), les caractéristiques de distribution pour un groupe sont déterminées en calculant la moyenne de la caractéristique de distribution du groupe $G_n$.

**7.** Procédé selon la revendication 6, dans lequel la moyenne est une moyenne pondérée.

**8.** Procédé selon l'une quelconque des revendications 1 à 7, dans lequel lesdites caractéristiques de distribution sont des valeurs d'écart type.

**9.** Procédé selon la revendication 8, comprenant en outre l'étape consistant à déterminer si une paire donnée de valeurs d'expression se trouve au sein d'un seuil significatif prédéterminé ou en dehors de celui-ci.

**10.** Procédé selon la revendication 9, comprenant également la présentation des variations négligeables par rapport aux variations importantes, dans l'expression génique, sur la base desdites caractéristiques de distribution calculées par segments.

Fig. 1

Fig. 2

Fig. 3

**Fig. 4**

Fig. 5

group genes of similar expression profile into slices (e.g. by means of rotation and subsequent taking equally populated groups)

compute statistics within slices

display resulting relative statistics in the original plot (e.g. colour coded for different statistical significance regions)

Fig. 6

Fig. 7

Fig. 8

Fig. 9

Legend:
- 1st SD region
- 2nd SD region, excluding 1st SD region
- 3rd SD region, excluding 2nd SD region
- 4th SD region, excluding 3rd SD region
- outside of 4th SD region

EP 1 907 962 B1

Fig. 10

EP 1 907 962 B1

Fig. 11

positive paired t-test (p=0.01)

EP 1 907 962 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0184139 A